**Europäisches Patentamt**

**European Patent Office**

**Office européen des brevets**

(11) Veröffentlichungsnummer: **0 119 596**
**A1**

# (12) EUROPÄISCHE PATENTANMELDUNG

(21) Anmeldenummer: **84102816.0**

(22) Anmeldetag: **15.03.84**

(51) Int. Cl.³: **A 61 M 25/00, A 61 M 5/00**

(30) Priorität: **18.03.83 DE 3309788**

(43) Veröffentlichungstag der Anmeldung: **26.09.84**
**Patentblatt 84/39**

(84) Benannte Vertragsstaaten: **AT BE CH DE FR GB IT LI LU NL SE**

(71) Anmelder: **Aigner, Karl, dr., Uhlandstrasse 5, D-6301 Pohlheim (DE)**

(72) Erfinder: **Aigner, Karl, dr., Uhlandstrasse 5, D-6301 Pohlheim (DE)**

(74) Vertreter: **Sternagel, Hans-Günther, Dr. et al, Patentanwälte Dr. M. Hann Dr. H.-G. Sternagel Marburger Strasse 38, D-6300 Giessen (DE)**

(54) **Implantierbarer Zuspritzkatheter.**

(57) Implantierbarer Zuspritzkatheter mit einem Katheterrohr 1 und einem an dessen anderen Ende angeordneten Zuspritzteil 2, das mit dem Katheterrohr 1 durch eine, an einem seitlich am Zuspritzteil 2 angerodneten Anschlußstutzen 3 angreifende Steckverbindung 4 verbunden ist. Die Oberfläche der elastischen Membran 5 und des Zuspritzteils 2 ist mit einem gewebskompatiblen, porösen Kunststoff, der das Einwachsen von Zellen begünstigt, beschichtet. An dem dem Zuspritzteil 2 abgewandten Ende des Katheterrohres 1 ist etwa 3 bis 5 mm vom Ende entfernt ein Außenwulst 8 angeordnet. Bei einer bevorzugten Ausführungsform ist am Katheterrohr 1 ein Rückschlagventil angeordnet.

Beschreibung:

Gegenstand der Erfindung ist ein implantierbarer Zuspritzkatheter, insbesondere eine gegenüber den bekannten Ausführungsformen verbesserte konstruktive Gestaltung, deren Gewebeverträglichkeit durch eine Oberflächenpräparation erhöht ist.

Für gewisse Therapien ist eine ständig zu wiederholende Direktzufuhr von Pharmazeutika oder Hilfsstoffen in Blutgefäße, beispielsweise Arterien, erforderlich. Um die wiederholte Punktierung der Gefäße zu vermeiden, wurde bereits vorgeschlagen, einen Katheter mit am anderen Ende angeschlossenen Zuspritzteil dauerhaft in ein Gefäß zu plazieren und auch das Zuspritzteil unter die Haut in den Körper zu implantieren. Die von einigen Firmen bisher angebotenen Ausführungsformen haben jedoch in der Praxis verschiedentliche Nachteile gezeigt. Insbesondere hat sich die Gewebeverträglichkeit des Zuspritzteils als unbefriedigend erwiesen. Der Körper bildet bei nicht oder wenig kompatiblen Werkstoffen um das Implantat eine Kapsel, die das ständige Injizieren erschwert. Die Membranen erweisen sich nach häufigem Durchstechen als nicht mehr ausreichend dicht. In einigen Fällen versagte auch die Fixierung der Spitze des Katheterrohres im Gefäß, so daß es nach Herausrutschen zu Blutungen mit unangenahmen Nebenwirkungen kam. Nachfolgende Injektionen treten dann in das Gewebe und verursachen dort Schäden.

Aufgabe der vorliegenden Erfindung ist es, eine verbesserte konstruktive Gestaltung eines implantierbaren Zuspritzkatheters zu schaffen, der außerdem eine bessere Körperverträglichkeit aufweist als die bisher bekannten Ausführungsformen.

Diese Aufgabe wird gelöst durch den implantierbaren Zuspritzkatheter gemäß den Patentansprüchen.

Die Unteransprüche richten sich auf bevorzugte Ausführungsformen des Zuspritzkatheters.

Der implantierbare Zuspritzkatheter weist ein Katheterrohr mit einem an dessen der Katheterspitze abgewandten Ende angeordneten Zuspritzteil auf. Das Zuspritzteil hat die Form einer runden, flachen Schale, deren Oberseite mit einer elastischen Membran dicht verschlossen ist. Es sind auch andere Formen möglich. Wesentlich ist, daß der obere Schalenrand so ausgebildet ist, daß eine möglichst große elastische Membranfläche als Einstichfläche zur Verfügung steht und der Randwulst etwas übersteht, um das Auffinden der Punktionsstelle durch Tasten zu erleichtern. An der Unterseite ist ein seitlich überstehender Schalenrand vorhanden. Das Kennzeichnende der erfindungsgemäßen Ausführungsform besteht darin, daß das Zuspritzteil mit dem Katheterrohr durch eine an einem seitlich am Zuspritzteil angeordneten Anschlußstutzen angreifende Steckverbindung auswechselbar verbunden ist.

Um die Gewebeverträglichkeit des Zuspritzteils zu verbessern, ist die Oberfläche des Zuspritzteils vollständig oder teilweise mit einer porösen Schicht aus besonders gewebeverträglichen Kunststoffen überzogen. Um ein Verwachsen zumindest der Anstechfläche, d.h. der elastischen Membran auf der Oberseite des Zuspritzteils zu ermöglichen, ist bei einer bevorzugten Ausführungsform der Erfindung die Oberfläche der elastischen Membran und die der Oberkante des Schalenrandes des Zuspritzteils mit der porösen Kunststoffschicht überzogen. Grundsätzlich ist auch eine Beschichtung der gesamten Oberfläche oder eine Beschichtung mit einem Film möglich, um die Gewebeverträglichkeit zu verbessern. Als geeignete gewebeverträgliche Kunststoffe haben sich erwiesen Polyacrylnitril, Polyamid, Polyurethan, Polyethylenglykolterephthalat oder Silikone bzw. Mischungen dieser Polymeren. Polyethylenglykolterephthalat oder eine Mischung aus Polyurethan und Silikon sind bei Verwendung von elastischen Membranen aus Naturkautschuk besonders bevorzugt, können aber auch bei anderen Membranmaterialien, z. B. Silikonkautschuk, eingesetzt werden.

Um ein Herausrutschen der Spitze des Katheterrohres aus dem Gefäß zu vermeiden, weist das dem Zuspritzteil abgewandte Ende des Katheterrohres in 3 mm bis 5 mm Abstand vom Ende der Katheterspitze einen Außenwulst auf. Vorzugsweise ist der Außenwulst etwa 4 mm vom Katheterende entfernt und erhöht den Außendurchmesser des Katheters von 1,5 bis 2 mm auf 3 bis 4 mm.

Bei einer weiteren Ausführungsform ist im oder am Katheterrohr ein Rückschlagventil angeordnet. Dieses ist vorzugsweise an der Katheterrohrspitze angeordnet.

Bei einer solchen Anordnung und Ausführungsform kann die Katheterrohrspitze geschlossen sein. Das Rohr weist zwei gegeneinander versetzte seitliche Öffnungen auf, über die ein elastischer Ventilschlauch aus geeignetem Material gezogen ist und diese verschließt. Durch den von innen wirkenden Injektionsdruck wird der Ventilschlauch gedehnt und die Flüssigkeit kann aus dem zur Spitze hin offenen Ventilschlauch austreten. Der hintere Teil des Ventilschlauches schließt sich direkt an den Außenwulst an und kann dort auch an der Oberfläche des Katheterrohres befestigt sein.
Im Ruhezustand verschließt der Ventilschlauch die
Öffnungen und läßt einen Austritt von Blut in das
Katheterrohr nicht zu.

Der Vorteil der erfindungsgemäßen Konstruktion besteht darin, daß der Außenwulst auf dem Katheterrohr
eine Arretierung der Katheterspitze im Gefäß ermöglicht und so die Gefahr des Herausrutschens
verhindert ist.     Durch die Ausbildung einer Steckverbindung zwischen Katheterrohr und Zuspritzteil ist
ein Austausch des Zuspritzteils bei etwaigem Versagen
nach vielen Punktionen möglich, ohne daß eine neue
Freilegung des tiefer im Körper liegenden Gefäßes
erforderlich ist, weil das Katheterrohr nicht ausgetauscht zu werden braucht.

Die Beschichtung des Zuspritzteils mit besonders geweberträglichen Kunststoffen, zumindest auf der Oberseite und der elastischen Kautschukmembran des Zuspritzteils, hat den Vorteil, daß diese Teile mit
dem Gewebe verwachsen und so eine zusätzliche Abdichtung der bei Injektionen jeweils zu durchstechenden Membran erreicht wird. Für den Fall, daß die
Latexmembran durch den wiederholten Einstich undicht
werden sollte, besteht ein geringeres Risiko des Austretens von Flüssigkeit, sowohl zurücklaufenden Bluts
als auch Injektats, aus dem Zuspritzteil in das Gewebe.
Durch den Einbau eines Rückschlagventils in das Katheterrohr und die festere Fixierung der Katheterspitze im Gefäß ist das Risiko des Austretens von
Blut aus dem Gefäß oder aus einem undichten Zuspritzteil erheblich verringert. Das Rückschlagventil verhindert ein Zurückfließen in den Katheter, wo es durch
Gerinnen zu Verstopfungen und Thrombenbildung kommen
kann.

Dies stellt einen nicht zu unterschätzenden Vorteil dar, weil Notfallsituationen dadurch außerordentlich selten werden.

Die Erfindung wird nun anhand der Figuren noch näher erläutert.

Figur 1 zeigt den Zuspritzkatheter in zusammengesteckter Form in einem Längsschnitt.

Figur 2 zeigt den Zuspritzkatheter von oben.

Figur 3 zeigt das Zuspritzteil und das hintere Ende des Katheterrohres in auseinandergezogenem Zustand in einem Seitenschnitt.

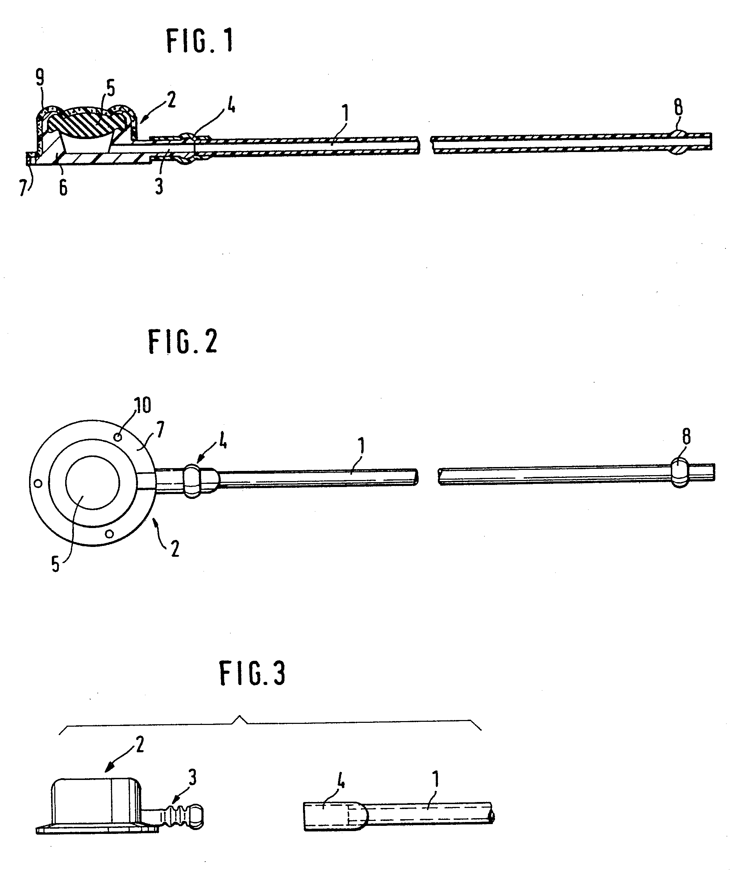

Aus Figur 1 ist ersichtlich, daß das Zuspritzteil 2 einen seitlichen Anschlußstutzen 3 aufweist, an den mittels der Steckverbindung 4 das Katheterrohr 1 angeschlossen ist. Das andere Ende des Katheterrohres 1 weist in geringem Abstand von dem Ende einen aufgesetzten Außenwulst 8 auf. Der Wulst 8 dient der Arretierung der Spitze im Gefäß. Ein Rückschlagventil ist in dieser Figur nicht wiedergegeben.

Das Zuspritzteil 2 hat die Form einer Dose oder flachen Schale 6, deren Oberseite mit einer elastischen Membran 5 verschlossen ist, die in den nach oben gerichteten Rand der Schale 6 dicht eingefügt ist. Zur besseren Fixierung des Zuspritzteils im Körper ist auf der Unterseite ein seitlich überstehender Rand 7 vorgesehen. Die zur Verbesserung der Gewebeverträglichkeit vorgesehene Beschichtung mit porösem Kunststoff ist in Figur 1 mit dem Bezugszeichen 9 angedeutet, wobei die Schichtdicke zur Verdeutlichung stark übertrieben ist. Das Katheterrohr hat vorzugsweise einen Innendurchmesser von 1,1 mm und einen Außendurchmesser von 1,7 mm. Der Außenwulst zur Arretierung weist bei einer bevorzugten Ausführungsform den Wulst in einem Abstand von 4 mm von der Spitze des Katheterrohres 1 auf. Das Gehäuse des Zuspritzteils, d. h. die Schale, kann aus geeigneten körperverträglichen Kunststoffen oder Metall hergestellt sein, vorzugsweise aus Polyoxymethylen. Die elastische Membran 5 ist üblicherweise ein dafür geeigneter Kautschuk, beispielsweise ein Silikonkautschuk oder Naturkautschuk.

Figur 2 zeigt den Zuspritzkatheter von oben, wobei der sich nach außen erstreckende seitliche Rand 7 mit Löchern 10 zum Fixieren mittels Fäden besonders deutlich zu erkennen ist. Im Zentrum des Zuspritzteils 2 auf der Oberseite liegt die Injektionsplatte in Form einer elastischen Membran 5. An den seitlich abführenden Stutzen ist mit der Steckverbindung 4 das Katheterrohr 1 angeschlossen, das an seinem anderen Ende den Arretierungswulst 8 aufweist.

Figur 3 zeigt das Zuspritzteil 2 in vom Katheterrohr 1 abgetrennten Zustand. Der seitliche Anschlußstutzen 3 am Zuspritzteil 2 weist an seiner Oberfläche Kerben und an seinem Ende einen Wulst auf, so daß das als übergreifende Hülse ausgebildete Ende des Katheterrohres 1 darüber geschoben werden kann und die Steckverbindung 4 bildet. Der Endwulst und die Rippen auf der Oberfläche des Stutzens 3 ergeben einen dichten, festen Sitz der Hülse des Katheterrohres und bewirken eine dauerhafte und dichte Verbindung, die jedoch im Bedarfsfalle leicht lösbar ist und das Anschließen eines neuen Zuspritzteils 2 an die Steckverbindung 4 ermöglicht. Das Katheterrohr wird vorzugsweise aus Silikonkautschuk oder Polyurethan hergestellt. Dieses Material ist ausreichend elastisch, um eine dichte Steckverbindung zu ermöglichen.

0119596

Bezugszeichenliste

1 Katheterrohr

2 Zuspritzteil

3 seitlicher Anschlußstutzen

4 Steckverbindung

5 elastische Membran

6 Schale des Zuspritzteils

7 seitlich überstehender Rand

8 Wulst

9 Beflockungsschicht

10 Befestigungslöcher

Patentansprüche

1. Implantierbarer Zuspritzkatheter mit einem Katheterrohr und einem an dessen anderem Ende angeordneten Zuspritzteil in Form einer runden Schale, deren Oberseite mit einer elastischen Membran als Injektionsplatte dicht verschlossen ist und die an der Unterseite einen seitlich überstehenden Rand aufweist,

d a d u r c h   g e k e n n z e i c h n e t ,

daß der obere Schalenrand des Zuspritzteils (2) als über die Membran (5) überstehender Randwulst ausgebildet ist und

daß das Zuspritzteil (2) mit dem Katheterrohr (1) durch eine, an einem seitlich am Zuspritzteil (2) angeordneten Anschlußstutzen angreifende Steckverbindung (4) verbunden ist.

2. Zuspritzkatheter nach Anspruch 1,

d a d u r c h   g e k e n n z e i c h n e t ,

daß die Oberfläche der elastischen Membran und des Zuspritzteils mit einem gewebskompatiblen, porösen Kunststoff, der das Einwachsen von Zellen begünstigt, beschichtet ist.

3. Zuspritzkatheter nach Ansprüchen 1 oder 2,

d a d u r c h   g e k e n n z e i c h n e t ,

daß an dem dem Zuspritzteil (2) abgewandten Ende des Katheterrohres etwa 3 bis 5 mm vom Ende entfernt ein Außenwulst (8) angeordnet ist.

4. Zuspritzkatheter nach Ansprüchen 1 oder 2,

d a d u r c h   g e k e n n z e i c h n e t ,

daß an dem dem Zuspritzteil abgewandten Ende des Katheterrohres etwa 4 mm vom Ende entfernt ein Außenwulst (8) angeordnet ist.

5. Zuspritzkatheter nach Ansprüchen 1 bis 4,
   d a d u r c h   g e k e n n z e i c h n e t ,
   daß im Katheterrohr (1) ein Rückschlagventil angordnet ist.

FIG.1

FIG.2

FIG.3

Europäisches
Patentamt

**EUROPÄISCHER RECHERCHENBERICHT**

0119596

Nummer der Anmeldung

EP  84 10 2816

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-3 971 376  (WICHTERLE) | | A 61 M   25/00<br>A 61 M    5/00 |
| A | US-A-4 226 334  (WEILER) | | |
| A | DE-C-  63 614  (DE PEZZER) | | |
| P | FR-A-2 513 128  (MEDTRONIC) | | |

RECHERCHIERTE
SACHGEBIETE (Int. Cl. ³)

A 61 M
B 65 D

Der vorliegende Recherchenbericht wurde für alle Patentansprüche erstellt.

| Recherchenort<br>DEN HAAG | Abschlußdatum der Recherche<br>25-05-1984 | Prüfer<br>VANRUNXT J.M.A. |
|---|---|---|